# EUROPEAN PATENT SPECIFICATION

(11) **EP 1 482 976 B1**
(45) Date of publication and mention of the grant of the patent: **20.08.2008**
(21) Application number: 03702672.1
(22) Date of filing: 12.02.2003
(51) Int. Cl.: A61K 45/00, A61K 31/195, A61K 31/4172, A61K 31/405, A61P 3/04

(54) **A PREPARATION AND METHOD FOR WEIGHT REDUCTION**
ZUBEREITUNG UND VERFAHREN ZUR GEWICHTSREDUKTION
PREPARATION ET PROCEDE POUR L'AMINCISSEMENT

(30) Priority: 20.02.2002 FI 20020332
(43) Date of publication of application: 08.12.2004
(73) Proprietor: Remedal Oy, 00200 Helsinki (FI)
(72) Inventor: HEINO, Pia, FIN-00200 Helsinki (FI)
(74) Representative: Laitinen, Pauli Sakari
(86) International application number: PCT/FI2003/000107
(87) International publication number: WO 2003/070276

(56) References cited:
- EP-A1- 0 301 122
- WO-A2-02/02103
- GB-A- 652 237
- US-A- 4 042 687
- US-A- 4 491 578
- US-A- 5 607 671
- DATABASE WPI Week 200259, Derwent Publications Ltd., London, GB; Class B04, AN 2002-552774, XP002966481 & JP 2002 154976 A (SANPO KK) 28 May 2002
- DATABASE WPI Week 199633, Derwent Publications Ltd., London, GB; Class B04, AN 1996-323071, XP002966482 & ES 2 087 027 A1 (UNIV ILLES BALEARS CTRA) 01 July 1996
- PATENT ABSTRACTS OF JAPAN vol. 14, no. 571 (C-790) 19 December 1990 & JP 02 250 823 A (TSUMURA & CO) 08 October 1990

## Description

This invention relates to weight reduction by taking with meals a formula in accordance with the invention.

A person weighing 70 kg consumes on average 10 MJ of energy per 24 hours. Of this energy, 45% is derived from carbohydrate, 20% from protein and 35% from fat. These figures correspond to approximately 260 g carbohydrate, 120 g protein and 90 g fat. It is also apparent that the energy content per unit weight of latter group of nutrients is more than twice that of the other two groups. After a meal, these energy-yielding nutrients are degraded by digestive enzymes to a form optimal for absorption from the gastrointestinal tract into the blood stream. On average, 85% of the carbohydrate, 95% of the fat and 80% of the protein is absorbed. Chained carbohydrates are degraded to monosaccharides, proteins are degraded to peptides and amino acids, and fats are degraded to free fatty acids and cholesterol. Apart from being a source of energy, nutrients are used by the body as structural components of tissues, cells, enzymes, antibodies, etc. Such use is almost exclusively limited to proteins. Proteins are composed of 20 L-amino acids. Eleven of these amino acids can be synthesised by the human body (nonessential amino acids), whereas nine amino acids are such that the body cannot produce (essential amino acids). The latter must therefore be obtained from the diet. The adult daily protein requirement is about 60 g, with essential amino acids accounting for about 9 g of this amount. In cases of insufficient protein intake from food, the liver is able to synthesise enough of all nonessential amino acids from carbohydrates and fats. If, however, there is a lack of essential amino acids in food, the affected person will develop malabsorption syndrome manifested as kwashiorkor. This disease is mainly encountered in developing countries.

If the amount of energy contained in absorbed food exceeds the energy consumption of the body, the excess will be stored in the body in the form of fat. In the industrialized world, where the population has access to a rich diet, this often leads to overweight with associated adverse effects on health.

It has proved difficult therapeutically to eliminate overweight or to reduce it to any significant extent. The available treatment options comprise eating less, following a low-calorie diet, energy consuming exercise and pharmacological therapy. Each of these alternatives requires that the patient is highly motivated to lose weight and receives continuous mental support in this effort. Even so, normal weight is often not attained, and any reasonably good result tends to be obliterated by a gradual return to original weight after the end of the treatment.

The currently known appetite suppressants, e.g. amfepramone, fenfluramine and sibutramine, are associated with side effects such as insomnia, blood pressure elevation and, in the case of the two first-mentioned drugs, also drug dependence. Fenfluramine, furthermore, has caused heart valve disease, leading to its withdrawal from the market in most countries. Treatment of obesity with orlistat, an inhibitor of fat absorption, calls for good motivation to lose weight and a low-fat diet. Orlistat, too, has specific side effects which limit its usefulness.

The present invention provides a remedy to the unsatisfactory state of affairs described above.

After a meal, the hepaticopancreatic duct, which empties into the duodenum somewhat distally from the pylorus, starts to discharge trypsinogen, an inactive proenzyme secreted by the pancreas. The wall of the duodenum contains an enzyme called enterokinase. Enterokinase converts the inactive trypsinogen to active trypsin which in turn breaks down the chyme-contained proteins into peptides. Trypsin further activates other enzymes responsible for the digestion of food proteins. Trypsin also activates any remaining trypsinogen through an autocatalytic mechanism. The 50 - 100 mg of trypsin secreted in conjunction with eating is able to break down into peptides all of the 30-60 g of protein contained in a normal meal. Without the action of trypsin, this digestion of protein will not take place.

The above situation occurs in certain pancreatic diseases, sometimes progressing to complete cessation of trypsin secretion. These patients have to take with meals an enzyme preparation (e.g. Pancreatin^{®}. Twin Laboratories Inc., Hauppauge, NY, USA) that digests food proteins and thus makes up for the lacking trypsin secretion.

The action of trypsin can be prevented by trypsin inhibitors, most of which are proteins by nature. In the presence of trypsin, these compounds bind to trypsin, forming inactive complexes. Well known trypsin inhibitors include alpha 1 -antitrypsin, trypstatin, alpha 2-macroglobulin, soybean trypsin inhibitors, egg-white trypsin inhibitors and aprotinin. Each of these is available commercially, and the latter substance is also registered as a medicinal product (e.g. Trasylol^{®}. Bayer AG, Leverkusen, Germany). Therapeutic oral administration of trypsin inhibitors and trypsin inactivation in the alimentary tract have been published for instance in patent documents JP 2-250823 (A) and US 5607671.

US 4491578 describes that the trypsin inhibitor aprotinin has been administered by the oral route in purpose of reducing satiety through its negative feedback signal for cholecystokinin secretion. In US 4042687 and ES 2087027 this result is pursued by different combinations of essential amino acids, which are added to the meals.

The present invention is aimed at preventing the absorption of proteins. This is achieved by oral administration of one, of the above-mentioned trypsin inhibitors with meals whereby the trypsin inhibitor moves along the alimentary tract to trypsin's site of action in the intestine. When for instance soybean trypsin inhibitor (Type II-S. Sigma Chemical Co., St. Louis, MO, USA) is used in accordance with the invention, one weight unit of the inhibitor can inactivate 2.5 weight units of trypsin secreted into the duodenum by the pancreas. As the action of trypsin is thus inhibited, the proteins contained in the food are not digested and cannot be absorbed. This results in artificial protein malabsorption which inevitably leads to loss of body weight.

Nevertheless, such usage of trypsin inhibitor alone causes not only weight loss but in the long run even malabsorption syndrome. The present invention, therefore, includes oral coadministration of required amounts of all essential amino acids in free form, together with the trypsin inhibitor. The essential amino acids are L-phenylalanine, L-histidine, L-isoleucine, L-leucine, L-lysine, L-methionine, L-threonine, L-tryptophan and L-valine. While weight is lost, these essential amino acids prevent the development of malabsorption syndrome. Of all amino acids, only the above-mentioned ones constitute a part of the invention. Since the nonessential amino acids do not function as required by the invention, they cannot by used as active substances in its embodiments. Free amino acids are fully absorbed from the alimentary tract, and their absorption is not affected by the trypsin inhibitor.

The present invention allows the amount of absorbed food protein and the energy therein to be reduced without causing a deficiency of essential amino acids. The result is weight reduction without disturbance of the nutritional status of the body.

For of the above-described effect to be achieved, the invention is characterized by the features presented in the patent claims.

As protein structure can be altered by the action of hydrochloric acid and pepsin in the stomach, the trypsin inhibitor is preferably transported to trypsin's site of action in the intestine in a formulation that does not release the active substance until it has passed the stomach. This is achieved by application of a "programmed delivery pattern" in the pharmaceutical formulation.

An applicable technique for this purpose is for instance microgranulation in combination with enteric coating which protects the active substance against the digestive effect of gastric juice. Enteric coatings suitable for embodiments of the invention include polyvinyl acetate (Colorcon^{®}. Colorcon Inc., West Point, PA, USA) and the acrylic acid derivatives described in patent document JP 2-250823 (A).

Said enteric coatings dissolve when the ambient pH exceeds 4.7. As such pH is characteristic of the intestine right from the beginning of the duodenum, release of the active substance is accomplished exactly in this desired location below the stomach. It then mixes with trypsin in the chyme, causing inhibition of trypsin activity.

The present invention affords the following novel and unexpected advantages over prior art:
- The person trying to lose weight does not have to change his/her habitual diet in any way even if this particular diet were the cause of the person's overweight.
- No changes are required in the daily amount of food ingested by the person, i.e. his/her daily intake of energy from food, even if the amount of energy contained in those very same meals were the cause of the person's overweight.

And still the person will lose weight.

The inventiveness of the present invention is further enhanced by the fact that
- it involves no effort to control the feeling of hunger or appetite of the person who is trying to lose weight
   and the fact that
- the person concerned is not required to subdue, limit or regulate his/her hunger or appetite in any way.

### Exemplifying embodiment

One dose of a preparation in accordance with the invention is as follows:

| | |
|---|---|
| Soybean trypsin inhibitor | 150 mg |
| L-Phenylalanine | 250 mg |
| L-Histidine | 200 mg |
| L-Isoleucine | 200 mg |
| L-Leucine | 250 mg |
| L-Lysine | 200 mg |
| L-Methionine | 200 mg |
| L-Threonine | 200 mg |
| L-Tryptophan | 150 mg |
| L-Valine | 200 mg |

Said dose (2 g) may be formulated, for instance, as a sachet or capsule containing a single-dose of powder or as a tablet containing the powder in combination with approved excipients or, for instance, an aqueous solution of approved excipients and the active ingredients.

Totally five doses (10 g) of the formula presented above are taken with daily meals in one of the pharmaceutical formulations presented above. This amount is sufficient and adequate to satisfy the daily requirement for all essential amino acids.

In accordance with the invention, any nontoxic compound that inactivates trypsin can be used for trypsin inhibition. Proteinous trypsin inhibitors can be enteric encapsulated, for instance, by coating microgranules with polyvinyl acetate (Colorcon^{®}). Such granules can be administered either on their own or, for instance, in a gelatine capsule together with essential amino acids, or the two entities can be taken as separate preparations, albeit during the same meal.

## Claims

1. An oral preparation for reducing weight, **characterised in that** the formula of the preparation contains simultaneously all the essential amino acids in free form and in addition a trypsin inhibitor selected from the group of alpha 1-antitrypsin, trypstatin, alpha 2-macroglobulin, soybean trypsin inhibitors, egg-white trypsin inhibitor and aprotinin, as active substances.

2. Use of a trypsin inhibitor and essential amino acids for producing an oral preparation according to claim 1 for reducing weight.

## Patentansprüche

1. Orales Präparat zur Gewichtsabnahme, **dadurch gekennzeichnet, dass** die Formel des Präparats gleichzeitig sämtliche essentiellen Aminosäuren in freier Form und zusätzlich einen Trypsininhibitor enthält, der aus der Gruppe von Alpha-1-Antitrypsin, Trypstatin, Alpha-2-Macroglobulin, Sojabohnen-Trypsininhibitoren, Eiweiss-Trypsininhibitor und Aprotinin als aktive Substanzen ausgewählt ist.

2. Verwendung von einem Trypsininhibitor und essentiellen Aminosäuren zur Produzierung eines oralen Präparats nach Patentanspruch 1 zur Gewichtsabnahme.

## Revendications

1. Préparation orale pour réduire le poids, **caractérisée en ce que** la formule de la préparation contient simultanément tous les acides aminés essentiels sous forme libre et en plus un inhibiteur trypsique sélectionné dans le groupe d'alpha 1 antitrypsine, de trypstatine, d'alpha 2 macroglobuline, d'inhibiteurs trypsiques du soja, d'inhibiteur trypsique de blanc d'oeuf et d'aprotinine, comme substance actives.

2. Utilisation d'un inhibiteur trypsique et d'acides aminés essentiels pour produire une préparation orale selon la revendication 1 pour réduire le poids.
